# EUROPÄISCHE PATENTSCHRIFT

(11) **EP 2 151 174 B1**
(45) Veröffentlichungstag und Bekanntmachung des Hinweises auf die Patenterteilung: **21.05.2014**
(21) Anmeldenummer: 08014173.2
(22) Anmeldetag: 08.08.2008
(51) Int. Cl.: A45D 34/04, A61F 13/38, A61M 35/00, A61F 15/00

(54) **Anordnung einer Mehrzahl von Wattestäbchen für kosmetische oder medizinische Zwecke**
Assembly of a number of cotton buds for cosmetic or medicinal uses
Dispositif doté d'une multitude de coton-tiges à usages cosmétiques ou médicaux

(43) Veröffentlichungstag der Anmeldung: 10.02.2010
(73) Patentinhaber: CMC Consumer Medical Care GmbH, 89567 Sontheim an der Brenz (DE)
(72) Erfinder: Mangold, Rainer, Dr., 89542 Herbrechtingen (DE); Albert, Susanne, 89233 Neu-Ulm (DE); Aupke, Michael, 41542 Dormagen (DE); Steiner, Katja-Leyla, 73572 Hechingen (DE)
(74) Vertreter: DREISS Patentanwälte PartG mbB

(56) Entgegenhaltungen:
- EP-A- 0 878 146
- WO-A-00/00147
- WO-A-2007/082353
- DE-U1- 20 020 066
- FR-A- 2 064 475
- US-A- 5 947 986

## Beschreibung

Die Erfindung betrifft eine Anordnung umfassend ein Verpackungsbehältnis und eine Mehrzahl von Wattestäbchen für kosmetische oder medizinische Zwecke in dem Verpackungsbehältnis mit einem eine innere Bodenfläche bildenden Bodenabschnitt, mit einem eine Entnahmeöffnung bildenden Wandabschnitt und mit einem das Verpackungsbehältnis in wesentlichen verdunstungshemmend verschließenden Deckel, wobei der Bodenabschnitt, der Wandabschnitt und vorzugsweise auch der Deckel aus einem formstabilen und feuchtigkeitsundurchlässigen Material ausgebildet sind und wobei die Wattestäbchen an ihren beiden Enden einen unverhüllten Wattekopf aus Fasermaterial aufweisen und im wesentlichen in vertikaler Ausrichtung in dem Verpackungsbehältnis aufgenommen sind, wenn das Verpackungabehältnis mit seinem Bodenabschnitt auf einer horizontalen Fläche abgestellt ist, und wobei jeweils der Wattestäbchen mit einer Befeuchtungsflüssigkeit befeuchtet ist und jeweils der dem Deckel zugewandte Wattekopf der Wattestäbchen unbefeuchtet ist.

Eine derartige Anordnung ist beispielsweise bekannt aus US-A-5,947,986. Die Lehre dieser Druckschrift besteht allerdings darin, die befeuchteten Watteköpfe der Wattestäbchen mit einer individuellen Umhüllung zu versehen, die ein Abtropfen von Befeuchtungsflüssigkeit verhindert und verdunstungshemmend wirkt. Nach einer Ausführungsform sind aber auch trockene Watteköpfe mit einer solchen Umhüllung versehen, um zu verhindern, dass Feuchtigkeit von außerhalb an die Watteköpfe gelangt. Nach einer weiteren Ausführungsform sind Wattestäbchen mit einem unverhüllten Wattekopf in einem feuchtigkeitsspeichernden Schwammmaterial am Boden des Verpackungsbehältnisses aufgenommen. Ihr jeweils gegenüberliegender oberer Wattekopf soll dabei trocken bleiben.

JP-A-2000 140013, offenbart ein Wattestäbchen, bei dem beide Watteköpfe mit einer auf Ölbasis beruhenden Befeuchtungsflüssigkeit beaufschlagt sind.

JP-A-2000 014588 offenbart ein trockenes Wattestäbchen, bei dem ein Ende schwarz oder fast schwarz eingefärbt ist, um eine bessere Kontrastierung für Ohrschmalz zur Verfügung zu stellen.

Um verschieden imprägnierte Wattestäbchen voneinander unterscheiden zu können, wurde mit FR-A-2.064.475 vorgeschlagen, deren Watteköpfe unterschiedlich einzufärben.

Der vorliegenden Erfindung liegt die Aufgabe zugrunde, Wattestäbchen der eingangs beschriebenen Art beziehungsweise eine Anordnung einer Mehrzahl solcher Wattestäbchen im Hinblick auf eine einfache und kostengünstige Herstellbarkeit und im Hinblick auf ihre praktische Verwendbarkeit zu verbessern.

Diese Aufgabe wird durch eine Anordnung mit den Merkmalen des Anspruchs 1 gelöst.

Dadurch dass die Wattestäbchen mit ihrem befeuchteten Wattekopf unmittelbar gegen die innere Bodenfläche des Bodenabschnitts anliegen, lässt sich die Befeuchtung der Watteköpfe ausführen, indem eine spezifische Menge an Befeuchtungsflüssigkeit in das Verpackungsbehältnis auf die innere Bodenfläche gegeben wird und dann die noch trockenen Wattestäbchen in das Verpackungsbehältnis eingestellt werden, wobei währenddessen das Verpackungsbehältnis vorzugsweise im Wesentlichen vertikal orientiert ist. Nach dem Einsetzen oder Einstellen der wattestäbchen nehmen die auf der inneren Bodenfläche aufstehenden Watteköpfe die Flüssigkeit am Boden des Verpackungsbehältnisses weitestgehend auf, so dass die Befeuchtungsflüssigkeit innerhalb des Verpackungsbehältnisses im Wesentlichen vollständig in den Watteköpfen der Wattestäbchen aufgenommen ist. Die Menge der Befeuchtungsflüssigkeit soll dabei so bemessen sein, dass nach einiger Zeit nach dem Einstellen der Wattestäbchen in das Verpackungsbehältnis im wesentlichen keine hin und her laufende Flüssigkeit mehr am Boden des Verpackungsbehältnisses vorhanden ist, wobei ein gewisser Feuchtigkeitsfilm an der inneren Bodenfläche wohl stets auf Grund von Oberflächeneffekten verbleiben wird. Dadurch dass die Befeuchtungsflüssigkeit auf Ölbasis beruht, können die dem Deckel zugewandten wattestäbchen auch während längerer Zeit der Lagerung des verschlossenen Verpackungsbehältnisses trocken gehalten werden, da die Befeuchtungsflüssigkeit im Unterschied zu wässriger Befeuchtungsflüssigkeit im Wesentlichen nicht in die Gasphase übergeht. Die Befeuchtungsflüssigkeit auf Ölbasis verbleibt also dauerhaft im Wesentlichen vollständig in den bestimmungsgemäß befeuchteten, gegen die innere Bodenfläche des Bodenabschnitts anliegenden Watteköpfen der Wattestäbchen, während die gegenüberliegenden Watteköpfe auch ohne eine Umhüllung zu verlangen, trocken bleiben.

Weiter kommt es beim Entnehmen der selektiv, also nur an einem Ende befeuchteten Wattestäbchen häufig zu Verwechslungen dahingehend, dass der Benutzer gerade nicht den intendierten Wattekopf zur Anwendung bringt, sondern den anderen. Es ist nämlich insbesondere für den flüchtigen Betrachter nicht ohne Weiteres erkennbar, welcher der Watteköpfe befeuchtet ist und welcher nicht. Der Benutzer neigt deshalb dazu, dies zuvor taktil zu prüfen, was aber nicht wünschenswert ist. Insofern erweist es sich als besonders vorteilhaft, dass die Watteköpfe auf der befeuchteten Seite der Wattestäbchen eine durch die Befeuchtungsflüssigkeit unlösliche Einfärbung des Fasermaterials aufweisen. Sofern beide Watteköpfe eines Wattestäbchen eingefärbt sind, so sollten sie unterschiedlich eingefärbt sein, damit sie voneinander unterschieden werden können. Auch im Hinblick auf die unlösliche Einfärbung erweist sich die Verwendung einer Befeuchtungsflüssigkeit auf Ölbasis als vorteilhaft. Wenn vorausgehend von einer Befeuchtungsflüssigkeit auf Ölbasis die Rede ist, so bedeutet dies, dass wenigstens 80 Gew.-% der Zusammensetzung der Befeuchtungsflüssigkeit von einem Öl gebildet sind. Vorzugsweise enthält die Befeuchtungsflüssigkeit wenigstens 85 Gew-%, weiter vorzugsweise wenigstens 90 Gew-%, insbesondere vorzugsweise wenigstens 95 Gew-% Öl.

Als Öle können dabei alle kosmetisch verwendbaren Öle eingesetzt werden. Öle sind dabei wasserunlösliche, organische Verbindungen, welche bei 20°C flüssig sind und einen relativ niedrigen Dampfdruck aufweisen.

Hierbei kommen vorzugsweise Mineralöle, wie beispielsweise Paraffinöl oder vaselinöl, insbesondere Paraffinum liquidum und Paraffinum perliquidum, pflanzliche Öle, wie beispielsweise Jojobaöl (flüssiger Wachsester), Süßmandelöl, Avocadoöl, Olivenöl, Palmöl, synthetische Öle, wie beispielsweise Silikonöle oder auch unter Normalbedingungen (1013mbar, 20°C) flüssige Fettalkohole in Frage. Insbesondere vorteilhaft sind Öle mit Ölkomponente auf Basis von Fettsäureestern aus Alkoholen mit 1 - 20 Kohlenstoffatomen und geradkettigen oder verzweigten Fettsäuren mit 3 - 24 Kohlenstoffatomen, wie beispielsweise Isocetylpalmitat. Insbesondere vorteilhaft sind Ester aus Glycerin mit Fettsäuren, insbesondere vorteilhaft sind Triacylglyceride mit Fettsäuren mit 3 - 24 Kohlenstoffatomen, insbesondere Triacylglyceride als gemischtsäurige Ester mit Caprylsäure (C8) und Caprinsäure (C10). Auch Mischungen von unterschiedlichen Ölen sind vorteilhaft.

Des Weiteren erweist es sich als vorteilhaft, wenn die Befeuchtungsflüssigkeit auf Ölbasis reinigende und/oder pflegende Wirkstoffe an sich bekannter Art und insbesondere in Form löslicher Extrakte umfasst. Diese Wirkstoffe können dabei vorzugsweise entnommen sein aus der Gruppe umfassend Vitamine, Antioxidantien, ätherische Öle, entzündungshemmende und/oder reizlindernde wirkstoffe. Emulgatoren können gegebenenfalls ebenso enthalten sein.

Bei der erfindungsgemäßen Anordnung beträgt die Anzahl der Wattestäbchen vorteilhafterweise 20 - 200, insbesondere 20 -150, insbesondere 20 - 120 , insbesondere 20 - 100, insbesondere 20 - 80, insbesondere 30 - 70, insbesondere 40 - 60 und vorzugsweise 50.

Im Hinblick auf eine weitgehende Aufnahme der Befeuchtungsflüssigkeit in dem Verpackungsbehältnis wird nach der Erfindung vorgeschlagen, dass die dem Verpackungsbehältnis zugegebene Menge an Befeuchtungsflüssigkeit 3 - 6 g und weiter insbesondere 4 - 5 g je 50 Stück Wattestäbchen beträgt. Es wird hierbei das Verständnis zugrundegelegt, dass bei einer von 50 Stück abweichenden vorliegenden Anzahl an Wattestäbchen, entsprechend auf die vorliegende Anzahl umgerechnet wird.

Insbesondere vorteilhaft ist, dass der Anteil an Befeuchtungsflüssigkeit 1,5 - 4,5 g, insbesondere 2 - 4 g bezogen auf 1 g trockenes Fasermaterial beträgt.

Für die Aufnahme der Befeuchtungsflüssigkeit durch die gegen die innere Bodenfläche anliegenden Watteköpfe erweist es sich als vorteilhaft, wenn die Befeuchtungsflüssigkeit niedrigviskos ist und hierfür vorzugsweise eine dynamische Viskosität η von 1 bis 250 mPas, weiter vorzugsweise von 1 - 200 mPas, insbesondere vorzugsweise von 1 - 150 mPas, weiter vorzugsweise von 1 - 100 mPas aufweist.

Die dynamische Viskosität η wird dabei aus der nach DIN 51 562 Teil 1 (Januar 1983) bei 20°C mit dem Ubbelohde-Viskosimeter gemessenen kinematischen Viskosität ν und unter Berücksichtigung der nach DIN 51757 (April 1994), Verfahren 4 gemessenen Dichte p (20°C) nach der Formel η = v x p ermittelt.

Vorteilhaft sind Befeuchtungsflüssigkeiten umfassend oder bestehend aus Paraffinöl (Paraffinum liquidum) als ölige Flüssigkeit, welches eine dynamische viskosität von 110-230 mPa·s aufweist. Insbesondere vorteilhaft sind Befeuchtungsflüssigkeiten umfassend oder bestehend aus dünnflüssigen Paraffinen (Paraffinum perliquidum), welches eine dynamische Viskosität von 25-80 mPa·s aufweist.

Insbesondere vorteilhaft ist die Befeuchtungsflüssigkeit umfassend oder bestehend aus Ölen, die eine dynamische viskosität von 1 - 100 mPas aufweisen.

Insbesondere vorteilhaft ist die Befeuchtungsflüssigkeit umfassend oder bestehend aus gemischtsäurigen Triacylglyceriden mit Caprylsäure (C8) und Caprinsäure (C10), welche eine dynamische Viskosität von 10 - 50 mPas aufweisen.

Vorteilhaft sind Befeuchtungsflüssigkeiten, welche einen Dampfdruck bei 20°C von kleiner 0,1 kPa, insbesondere kleiner 0,01 kPa, weiter insbesondere kleiner 0,001 kPa, weiter insbesondere kleiner 0,0001 kPa aufweisen.

Es erweist sich weiter als vorteilhaft, dass ein jeweiliger Wattekopf der Wattestäbchen, zumindest der jeweilige befeuchtete wattekopf der Wattestäbchen (jedoch im noch trockenen Zustand) 0,02 bis 0,05 g Fasermaterial umfasst. Die Länge eines jeweiligen Wattekopfs, gemessen in Längsrichtung eines Stiels der Wattestäbchen beträgt vorzugsweise 10 bis 35 mm, insbesondere 11 bis 16 mm. Der Durchmesser eines jeweiligen Wattekopfs, gemessen quer zur Längsrichtung des Stiels der Wattestäbchen, beträgt vorzugsweise 3 bis 12 mm, insbesondere 3,5 bis 5,5 mm.

Der Stiel des Wattestäbchens ist vorzugsweise aus Kunststoff, vorzugsweise umfassend Polypropylen.

Vorteilhaft ist, wenn der Wattekopf aus einem Faservlies oder einem Faservliesstreifen eines spezifischen Gewichts von 0,5 - 8 g/m, insbesondere 1 - 2 g/m hergestellt ist. Dabei weist das Faservlies oder der Faservliesstreifen insbesondere eine Breite von 10 - 20 mm, vorzugsweise von 12 - 18 mm auf.

Insbesondere vorteilhaft ist, wenn das Faservlies oder der Faservliesstreifen ein Kardenvlies mit in Maschinenrichtung verlaufender Orientierung ist.

Das Fasermaterial der Wattestäbchen besteht dabei vorzugsweise aus Baumwollfasern, oder es umfasst Baumwollfasern. Bevorzugt wird Fasermaterial, welches Mischungen aus Baumwollfasern und synthetischen Stapelfasern, insbesondere Mikrostapelfasern, oder Viskose-Stapelfasern umfasst.

Unter "Mikrostapelfasern" sind synthetische Fasern einer Faserstärke von ≤ 1 dtex zu verstehen. Des Weiteren bringt der Begriff Mikrostapelfaser auch zum Ausdruck, dass für die Herstellung des band- oder schnurförmigen Faservlieses zuvor in einem separaten Herstellungsverfahren gebildete Mikrofasern einer bestimmten Länge oder eines bestimmten Längenbereiches verwendet werden.

Nach einer bevorzugten Zusammensetzung des Fasermaterials umfasst dieses zu 3 bis 50 Gew.-%, insbesondere zu 5 bis 30 Gew.-% und vorzugsweise zu 5 bis 20 Gew.-% Mikrostapelfasern einer Länge von wenigstens 7 mm und einer Stärke von ≤ 1 dtex, vorzugsweise von etwa 0,9 dtex. Ferner umfasst das Fasermaterial bis zu 97 Gew.-%, insbesondere 60 bis 95 Gew.-% und vorzugsweise 70 bis 95 Gew.-% Baumwolle. Bei den vorstehend erwähnten Mikrostapelfasern handelt es sich um Polyester oder Viskosefasern mit einer Faserlänge von 18 bis 38 mm. Bevorzugtermaßen umfasst das Fasermaterial 90 Gew.-% Baumwollfasern, vorzugsweise Baumwollkämmlinge, und 10 Gew.-% Mikrostapelfasern.

Die Einfärbung des Fasermaterials wird vorteilhafterweise bereits im Herstellungsprozess des Fasermaterials durchgeführt. Die Einfärbung dient dem Zweck, die Watteköpfe voneinander unterscheiden zu können, so dass der Benutzer stets sicher weiß, welcher Wattekopf eines Wattestäbchens befeuchtet ist und welcher trocken ist.

Eine besonders bevorzugte Ausführungsform ist eine erfindungsgemäße Anordnung mit 20 - 120 Wattestäbchen, insbesondere vorzugsweise mit 50 Wattestäbchen, mit Watteköpfen mit 0,02 - 0,05 g Fasermaterial bestehend aus Baumwollfasern. Die Befeuchtungsflüssigkeit umfasst dünnflüssiges Paraffin (Paraffinum perliquidum) mit einer dynamischen Viskosität von 25-80 mPa·s. Vorteilhaft werden 3 - 6 g der Befeuchtungsflüssigkeit pro 50 Wattestäbchen vorgelegt, derart dass der gegen die Bodenfläche anliegende Wattekopf 2-4 g Befeuchtungsflüssigkeit pro 1 g Fasermaterial umfasst. Die Befeuchtungsflüssigkeit enthält zudem noch Anteile an Wirkstoffen.

Eine weitere bevorzugte Ausführungsform der erfindungsgemäßen Anordnung umfasst 20 - 120 Wattestäbchen, insbesondere vorzugsweise mit 50 Wattestäbchen, mit Watteköpfen mit 0,02 - 0,05 g Fasermaterial bestehend aus Baumwollfasern. Die Befeuchtungsflüssigkeit basiert auf Triacylglyceriden, insbesondere auf gemischtsäurigen Triacylglyceriden mit Caprylsäure (C8) und Caprinsäure (C10), insbesondere mit einer dynamischen Viskosität von 10-50 mPa·s. Vorteilhaft werden 3 - 6 g der Befeuchtungsflüssigkeit pro 50 Wattestäbchen vorgelegt werden, derart dass der Wattekopf 2 - 4 g Befeuchtungsflüssigkeit pro 1 g Fasermaterial umfasst.

Eine weitere bevorzugte Ausführungsform der erfindungsgemäßen Anordnung umfasst 20 - 120 Wattestäbchen, insbesondere vorzugsweise mit 50 Wattestäbchen, mit Watteköpfen mit 0,02 - 0,05 g Fasermaterial bestehend aus Baumwollfasern. Die Befeuchtungsflüssigkeit basiert auf Isocetylpalmitat, insbesondere mit einer dynamischen Viskosität von 10-50 mPa·s. Vorteilhaft werden 3 - 6 g der Befeuchtungsflüssigkeit pro 50 Wattestäbchen vorgelegt werden, derart dass der Wattekopf 2 - 4 g Befeuchtungsflüssigkeit pro 1 g Fasermaterial umfasst.

Der eingangs erwähnte Deckel des Verpackungsbehältnisses könnte nach einer weniger bevorzugten Ausführungsform der erfindungsgemäßen Anordnung beispielsweise auch von einer insbesondere aufgesiegelten oder aufgeklebten Folienabdeckung gebildet sein.

Gegenstand der vorliegenden Erfindung ist auch ein Verfahren zum Herstellen einer vorstehend beschriebenen erfindungsgemäßen Anordnung von Wattestäbchen in einem Verpackungsbehältnis mit den Merkmalen des Anspruchs 12.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform der erfindungsgemäßen Anordnung und ihrer Herstellung. In der Zeichnung zeigt:
Figur 1 eine zeichnerische Darstellung einer erfindungsgemäßen Anordnung von Wattestäbchen in einem Verpackungsbehältnis mit abgenommenem Deckel des Verpackungsbehältnisses und
Figur 2 verdeutlicht die Abfolge der Herstellung der Anordnung nach Figur 1.

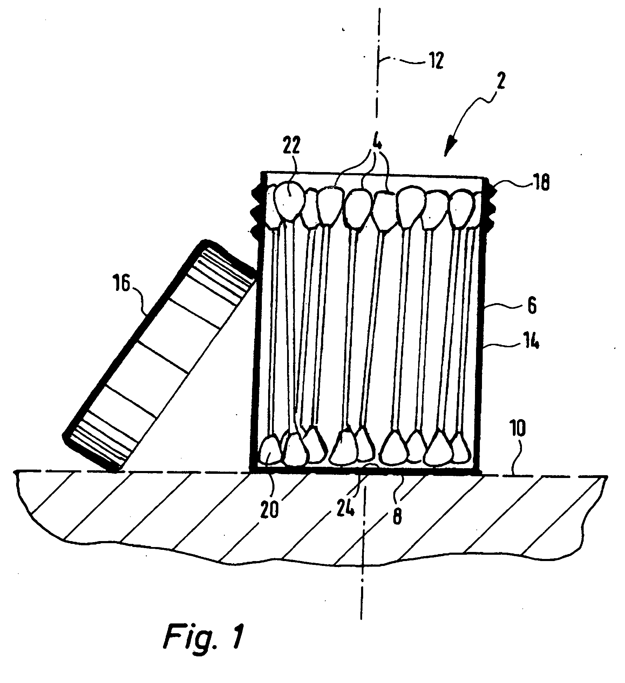

Figur 1 zeigt eine insgesamt mit dem Bezugszeichen 2 bezeichnete Anordnung einer Mehrzahl von Wattestäbchen 4 für kosmetische oder medizinische Zwecke in einem Verpackungsbehältnis 6. Das Verpackungsbehältnis 6 umfasst einen Bodenabschnitt 8, mit dem das Verpackungsbehältnis 6 auf einer vorzugsweise ebenen und horizontalen Fläche 10 abstellbar ist. Es weist dabei eine Hauptachse 12 in vertikaler Orientierung auf. An den Bodenabschnitt 8 schließt sich ein im beispielhaft dargestellten Fall zylindrischer Wandabschnitt 14 an, so dass das Verpackungsbehältnis 6 insgesamt topfförmig ausgebildet ist. Es ist an seinem oberen Ende durch einen Deckel 16 verschließbar, wobei im beispielhaft dargestellten Fall ein Gewindeverschluss vorgesehen ist und der Wandabschnitt 6 an seinem oberen Ende einen Außengewindeabschnitt 18 trägt. Das Verpackungsbehältnis 6 und seine Komponenten bestehen vorzugsweise aus einem flüssigkeitsdichten und auch feuchtigkeitsundurchlässigen Material, welches zwar in gewisser Weise nachgiebig biegsam, jedoch aber insgesamt formstabil, etwa im Unterschied zu einer Plastikfolienverpackung von Wattestäbchen, ausgebildet ist.

Das Verpackungsbehältnis 6 ist derart ausgebildet und an die Länge und Anzahl der Wattestäbchen 4 angepasst, dass die Wattestäbchen 4 wie zeichnerisch angedeutet in einer im Wesentlichen entlang der Hauptachse 12 ausgerichteten Orientierung in dem Verpackungsbehältnis 6 aufgenommen sind. Die Wattestäbchen 4 umfassen an ihren beiden Enden je einen Wattekopf 20 beziehungsweise 22. Mit ihrem dem Bodenabschnitt 8 des Verpackungsbehältnisses 6 zugewandten Wattekopf 20 liegen die Wattestäbchen unmittelbar gegen eine innere Bodenfläche 24 des Verpackungsbehältnisses 6 an. Diese innere Bodenfläche ist von dem formstabilen Material des Bodenabschnitts 8 des Verpackungsbehältnisses 6 gebildet. Nur diese dem Bodenabschnitt 8 zugewandten Watteköpfe 20 der Wattestäbchen 4 sind befeuchtet, während die gegenüberliegenden Watteköpfe 22 trocken bleiben, und zwar auch während längerer Lagerung des mittels des Deckels 16 verschlossenen Verpackungsbehältnisses 6. Als Befeuchtungsflüssigkeit für die Watteköpfe 20 wird nämlich eine zuvor entsprechend der Anzahl und Masse des Fasermaterials der Watteköpfe 20 zugemessene Menge an Befeuchtungsflüssigkeit auf Ölbasis verwendet, die in weitaus geringerem Maße zur Verdunstung neigt als wässrige Flüssigkeiten. Durch die zugemessene Menge an Befeuchtungsflüssigkeit wird erfindungsgemäß auch erreicht, dass die Befeuchtungsflüssigkeit durch das Fasermaterial der Watteköpfe 20 der Wattestäbchen 4 weitestgehend, das heißt im Wesentlichen vollständig (bis auf einen dünnen auf Oberflächeneffekte zurückzuführenden Film an der inneren Bodenfläche 24) aufgenommen wird. Auf diese Weise wird auch ausgeschlossen, dass bei Neigung der Verpackungsbehältnisse 6, insbesondere im Zuge des unkontrollierten Transports in geschlossenen Umverpackungen Flüssigkeit innerhalb des Verpackungsbehältnisses 6 zu fließen beginnt. Die Flüssigkeit ist vielmehr dauerhaft in den Watteköpfen 20 der Wattestäbchen 4 gespeichert, wenn die Anordnung herstellerseitig fertiggestellt ist und das Verpackungsbehältnis 6 mittels des Deckels 16 verschlossen ist.

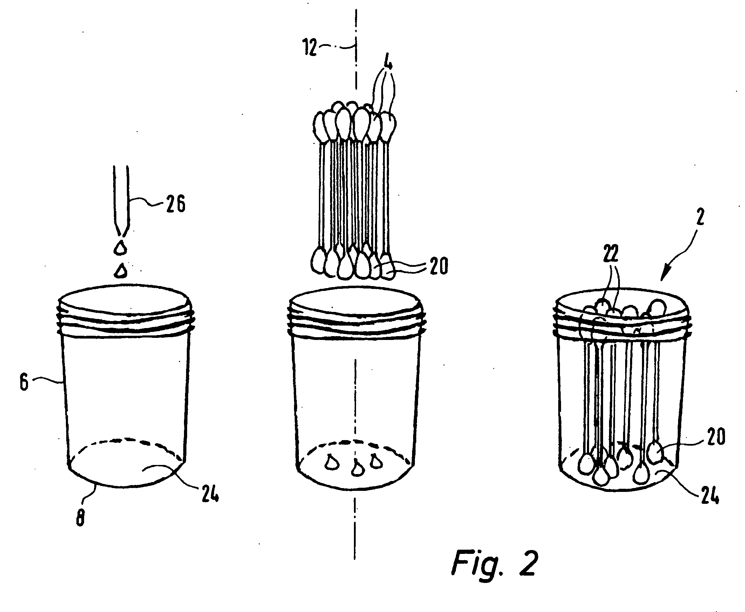

Figur 2 verdeutlicht die Herstellung der vorbeschriebenen Anordnung 2 in schematischer Weise, wobei der Deckel des Verpackungsbehältnisses 6 weggelassen ist. Wie in Figur 2 links dargestellt wird zunächst in das leere Verpackungsbehältnis 6 mittels einer geeigneten Vorrichtung 26, die hier nur schematisch dargestellt ist, eine vorgegebene Menge an Befeuchtungsflüssigkeit auf Ölbasis eingegeben, die sich dann auf der inneren Bodenfläche 24 des Verpackungsbehältnisses 6 sammelt. Sodann wird die Mehrzahl von Wattestäbchen 4 in das Verpackungsbehältnis 6 in der dargestellten Ausrichtung entlang der Hauptachse 12 des Verpackungsbehältnisses in das Verpackungsbehältnis eingestellt, so dass die dem Bodenabschnitt 8 zugewandten Watteköpfe 20 der Wattestäbchen 4 unmittelbar gegen die innere Bodenfläche 24 anliegen so wie dies in der rechten Darstellung der Figur 2 dargestellt ist. Das Fasermaterial der Watteköpfe 20 gelangt so in Kontakt mit der Befeuchtungsflüssigkeit auf Ölbasis und vermag diese in verhältnismäßig kurzer Zeit im Wesentlichen vollständig aufzunehmen. Während dieses Zeitraums der Herstellung der erfindungsgemäßen Anordnung 2 wird das Verpackungsbehältnis 6 weiter in vertikaler Orientierung geführt oder gehalten bis der Absorptionsprozess im Wesentlichen abgeschlossen ist. Die den Watteköpfen 20 gegenüberliegenden Watteköpfe 22 der Wattestäbchen 4 bleiben dauerhaft trocken, auch wenn die Anordnung 2 längere Zeit in feuchtigkeitsdichtem Zustand verschlossen bleibt.

## Patentansprüche

1. Anordnung (2) umfassend ein Verpackungsbehältnis (6) und eine Mehrzahl von Wattestäbchen (4) für kosmetische oder medizinische Zwecke in dem Verpackungsbehältnis (6) mit einem eine innere Bodenfläche (24) bildenden Bodenabschnitt (8), mit einem eine Entnahmeöffnung bildenden wandabschnitt (14) und mit einem das Verpackungsbehältnis (6) im wesentlichen verdunstungshemmend verschließenden Deckel (16), wobei der Bodenabschnitt (8), der Wandabschnitt (14) und vorzugsweise auch der Deckel (16) aus einem formstabilen und feuchtigkeitsundurchlässigen Material ausgebildet sind und wobei die Wattestäbchen (4) an ihren beiden Enden einen unverhüllten Wattekopf (20, 22) aus Fasermaterial aufweisen und im wesentlichen in vertikaler Ausrichtung in dem Verpackungsbehältnis (6) aufgenommen sind, wenn das Verpackungsbehältnis (E) mit seinem Bodenabschnitt (8) auf einer horizontalen Fläche (10) abgestellt ist, und wobei jeweils der der inneren Bodenfläche (24) zugewandte Wattekopf (20) der Wattestäbchen (4) mit einer Befeuchtungsflüssigkeit befeuchtet ist und jeweils der dem Deckel (16) zugewandte Wattekopf (22) der Wattestäbchen (4) unbefeuchtet ist, und die Wattestäbchen (4) mit ihrem befeuchteten Wattekopf (20) unmittelbar gegen die innere Bodenfläche (24) des Bodenabschnitts (8) anliegen, und die Befeuchtungsflüssigkeit innerhalb des Verpackungsbehältnisses (6) auf Ölbasis beruht und im wesentlichen vollständig in den gegen die innere Bodenfläche (24) anliegenden Watteköpfen (20) der Wattestäbchen (4) aufgenommen ist, **dadurch gekennzeichnet dass** die Menge an Befeuchtungsflüssigkeit innerhalb des Verpackungsbehältnisses (6) 3 - 6 g je 50 Stück Wattestäbchen (4) beträgt, dass ein jeweiliger Wattekopf (20, 22) 0,02 bis 0,05 g Fasermaterial und dass der Anteil an Befeuchtungsflüssigkeit 1,5 - 4,5g bezogen auf 1g trockenes Fasermaterial beträgt und dass die gegen die innere Bodenfläche (24) anliegenden Watteköpfe (20) zur Unterscheidung von den dem Deckel (16) zugewandten Watteköpfen (22) eine Markierung in Form einer durch die Befeuchtungsflüssigkeit unlöslichen Einfärbung des Fasermaterials dieser Watteköpfe (20) aufweisen.

2. Anordnung nach Anspruch 1, **dadurch gekennzeichnet, dass** die Anzahl der Wattestäbchen (4) 20 - 200, insbesondere 20 -150, insbesondere 20 - 120, insbesondere 20 - 100, insbesondere 20 - 80, insbesondere 30 - 70, insbesondere 40 - 60 und vorzugsweise 50 beträgt.

3. Anordnung nach Anspruch 1 oder 2, **dadurch gekennzeichnet, dass** die Menge an Befeuchtungsflüssigkeit innerhalb des Verpackungsbehältnisses (6) 4 - 5 g je 50 Stück Wattestäbchen (4) beträgt.

4. Anordnung nach einem oder mehreren der vorstehenden Ansprüche, **dadurch gekennzeichnet, dass** die Befeuchtungsflüssigkeit eine dynamische Viskosität von 1 - 250 mPas aufweist.

5. Anordnung nach einem oder mehreren der vorstehenden Ansprüche, **dadurch gekennzeichnet, dass** die Länge eines jeweiligen Wattekopfs, gemessen in Längsrichtung eines Stiels der Wattestäbchen (4) 10 bis 35 mm, insbesondere 11 bis 16 mm beträgt.

6. Anordnung nach einem oder mehreren der vorstehenden Ansprüche, **dadurch gekennzeichnet, dass** der Durchmesser eines jeweiligen Wattekopfs (20, 22), gemessen quer zur Längsrichtung des Stiels der Wattestäbchen (4), 3 bis 12 mm, insbesondere 3,5 bis 5,5 mm beträgt.

7. Anordnung nach einem oder mehreren der vorstehenden Ansprüche, **dadurch gekennzeichnet, dass** das Fasermaterial aus Baumwollfasern besteht oder Baumwollfasern umfasst.

8. Anordnung nach Anspruch 7, **dadurch gekennzeichnet, dass** das Fasermaterial Mischungen aus Baumwollfasern und synthetischen Stapelfasern oder Viskose-Stapelfasern umfasst.

9. Anordnung nach einem oder mehreren der vorstehenden Ansprüche, **dadurch gekennzeichnet, dass** der Wattekopf hergestellt ist aus einem Faservlies oder einem Faservliesstreifen eines spezifischen Gewichts von 0,5 - 8 g/m, insbesondere 1 - 2 g/m.

10. Anordnung nach Anspruch 9, **dadurch gekennzeichnet, dass** das Faservlies oder der Faservliesstreifen ein Kardenvlies mit in Maschinenrichtung verlaufender Orientierung ist.

11. Verfahren zum Herstellen einer Anordnung nach einem oder mehreren der vorstehenden Ansprüche, die folgenden Verfahrensschritte umfassend:
- Bereitstellen des Verpackungsbehältnisses (6),
- Zumessen einer vorgegebenen Menge an Befeuchtungsflüssigkeit auf die innere Bodenfläche (24) des Verpackungsbehältnisses (6),
- Bereitstellen der Mehrzahl von Wattestäbchen (4) mit einer Markierung in Form einer durch die Befeuchtungsflüssigkeit unlöslichen Einfärbung des Fasermaterials nur eines (20) der Watteköpfe (20, 22),
- Einstellen der Wattestäbchen (4) in das Verpackungsbehältnis (6) derart, dass die markierten Watteköpfe (20) unmittelbar gegen die innere Bodenfläche (24) abgestützt sind und
- Verschließen des Verpackungsbehältnisses (6) durch den Deckel (16).

12. Verfahren nach Anspruch 11, **dadurch gekennzeichnet, dass** die zugemessene Menge an Befeuchtungsflüssigkeit 2,5 - 6,5 g, insbesondere 3 - 6 g und weiter insbesondere 4 - 5 g Flüssigkeit je 50 Stück Wattestäbchen beträgt.

## Claims

1. An assembly (2) including a packaging container (6) and a plurality of cotton swabs (4) for cosmetic or medical purposes in the packaging container (6) having a bottom portion (8) forming an inner bottom face (24), having a wall portion (14) forming a removal opening, and having a cap (16) closing off the packaging container (6) in an essentially vapor-inhibiting manner, in which the bottom portion (8), the wall portion (14) and preferably also the cap (16) are embodied of a dimensionally stable, moisture-impermeable material, and the cotton swabs (4) have an unsheathed cotton tip (20, 22) of fiber material on both of their ends and are received in the packaging container (6) in an essentially vertical orientation, if the packaging container (6) is set down with its bottom portion (8) on a horizontal surface (10), and each cotton tip (20) of the cotton swabs (4) that is facing the inner bottom face (24) is moistened with a moistening fluid, and each cotton tip (22) of the cotton swabs (4) facing toward the cap (16) is unmoistened, and the cotton swabs (4) rest with their moistened cotton tip (20) directly on the inner bottom face (24) of the bottom portion (8), and the moistening fluid inside the packaging container (6) is oil-based and is received essentially entirely in the cotton tips (20), resting on the inner bottom face (24), of the cotton swabs (4), **characterized in that** the quantity of moistening fluid inside the packaging container (6) amounts to from 3 to 6 g per 50 cotton swabs (4); that each cotton tip (20, 22) includes from 0.02 to 0.05 g of fiber material; and that the proportion of moistening fluid amounts to from 1.5 to 4.5 g, referred to 1 g of dry fiber material; and that the cotton tips (20) resting on the inner bottom face (24), in order to be distinguishable from the cotton tips (22) facing toward the cap (16), have a marking in the form of a dye of the fiber material of these cotton tips (20) that is insoluble in the moistening fluid.

2. The assembly of claim 1, **characterized in that** the number of cotton swabs (4) is from 20 to 200, in particular 20 to 150, in particular 20 to 120, in particular 20 to 100, in particular 20 to 80, in particular 30 to 70, in particular 40 to 60, and preferably 50.

3. The assembly of claim 1 or 2, **characterized in that** the quantity of moistening fluid inside the packaging container (6) amounts to from 4 to 5 g per 50 cotton swabs (4).

4. The assembly of one or more of the foregoing claims, **characterized in that** the moistening fluid has a dynamic viscosity of 1 to 250 mPas.

5. The assembly of one or more of the foregoing claims, **characterized in that** the length of each cotton tip, measured in the longitudinal direction of a stem of the cotton swabs (4), amounts to from 10 to 35 mm, in particular from 11 to 16 mm.

6. The assembly of one or more of the foregoing claims, **characterized in that** the diameter of each cotton tip (20, 22), measured crosswise to the longitudinal direction of the stem of the cotton swabs (4) amounts to from 3 to 12 mm, in particular from 3.5 to 5.5 mm.

7. The assembly of one or more of the foregoing claims, **characterized in that** the fiber material comprises cotton fibers or includes cotton fibers.

8. The assembly of claim 7, **characterized in that** the fiber material includes mixtures of cotton fibers and synthetic staple fibers or viscose staple fibers.

9. The assembly of one or more of the foregoing claims, **characterized in that** the cotton tip is produced from a nonwoven fabric or a nonwoven fabric strip having a specific weight of from 0.5 to 8 g/m, in particular 1 to 2 g/m.

10. The assembly of claim 9, **characterized in that** the nonwoven fabric or the nonwoven fabric strip is a card web with an orientation in the machine direction.

11. A method for producing an assembly of one or more of the foregoing claims, including the following method steps:
- furnishing the packaging container (6),
- apportioning a predetermined quantity of moistening fluid onto the inner bottom face (24) of the packaging container (6),
- furnishing the plurality of cotton swabs (4) with a marking in the form of a dye, of the fiber material of only one (20) of the cotton tips (20, 22), that is insoluble in the moistening fluid;
- placing the cotton swabs (4) in the packaging container (6) in such a way that the marked cotton tips (20) are braced directly on the inner bottom face (24), and
- closing the packaging container (6) by means of the cap (16).

12. The method of claim 11, **characterized in that** the apportioned quantity of moistening fluid amounts to from 2.5 to 6.5 g, in particular from 3 to 6 g, and more particularly from 4 to 5 g of fluid per 50 cotton swabs.

## Revendications

1. Ensemble (2) comprenant un récipient d'emballage (6) et une pluralité de bâtonnets ouatés (4) à usage cosmétique ou médical dans ledit récipient d'emballage (6) ayant une portion de fond (8) formant une surface de fond (24) intérieure, une portion de paroi (14) formant une ouverture de prélèvement ainsi qu'un couvercle (16) fermant ledit récipient d'emballage (6) de manière à protéger pour l'essentiel contre l'évaporation, ladite portion de fond (8), ladite portion de paroi (14) et, de préférence, également ledit couvercle (16) étant réalisés dans une matière de forme stable et imperméable à l'humidité, et lesdits bâtonnets ouatés (4) présentant à leurs deux extrémités une tête ouatée non couverte (20, 22) en matière fibreuse et étant reçus pour l'essentiel dans une orientation verticale à l'intérieur du récipient d'emballage (6) lorsque ledit récipient d'emballage (6) est posé par sa portion de fond (8) sur une surface horizontale (10), et respectivement la tête ouatée (20) des bâtonnets ouatés (4) qui est tournée vers la surface de fond (24) intérieure étant mouillée d'un liquide de mouillage et respectivement la tête ouatée (22) des bâtonnets ouatés (4) qui est tournée vers ledit couvercle (16) est non mouillée, et lesdits bâtonnets ouatés (4) s'appliquant par leur tête ouatée (20) mouillée directement contre la surface intérieure de fond (24) de la portion de fond (8), et le liquide de mouillage à l'intérieur du récipient d'emballage (6) étant à base d'huile et étant absorbé pour l'essentiel complètement dans les têtes ouatées (20) des bâtonnets ouatés (4), qui s'appliquent contre la surface intérieure de fond (24), **caractérisé par le fait que** la quantité de liquide de mouillage à l'intérieur du récipient d'emballage (6) est comprise entre 3 et 6 g par 50 pièces de bâtonnets ouatés (4), qu'une tête ouatée (20, 22) respective comprend de 0,02 à 0,05 g de matière fibreuse et que la proportion de liquide de mouillage est comprise entre 1,5 et 4,5 g par rapport à 1 g de matière fibreuse sèche et que les têtes ouatées (20) s'appliquant contre la surface intérieure de fond (24) présentent, pour les distinguer de têtes ouatées (22) montrant vers le couvercle (16), un marquage sous forme d'une coloration de la matière fibreuse de ces têtes ouatées (20), qui est insoluble par le liquide de mouillage.

2. Ensemble selon la revendication 1, **caractérisé par le fait que** le nombre des bâtonnets ouatés (4) est compris entre 20 et 200, en particulier entre 20 et 150, en particulier entre 20 et 120, en particulier entre 20 et 100, en particulier entre 20 et 80, en particulier entre 30 et 70, en particulier entre 40 et 60 et est, de préférence, de 50.

3. Ensemble selon la revendication 1 ou 2, **caractérisé par le fait que** la quantité de liquide de mouillage à l'intérieur du récipient d'emballage (6) est comprise entre 4 et 5 g par 50 pièces de bâtonnets ouatés (4).

4. Ensemble selon l'une ou plusieurs des revendications précédentes, **caractérisé par le fait que** le liquide de mouillage présente une viscosité dynamique comprise entre 1 et 250 mPas.

5. Ensemble selon l'une ou plusieurs des revendications précédentes, **caractérisé par le fait que** la longueur d'une tête ouatée respective, mesurée dans la direction longitudinale d'une tige des bâtonnets ouatés (4), est comprise entre 10 et 35 mm, en particulier entre 11 et 16 mm.

6. Ensemble selon l'une ou plusieurs des revendications précédentes, **caractérisé par le fait que** le diamètre d'une tête ouatée (20, 22) respective, mesuré transversalement à la direction longitudinale de la tige des bâtonnets ouatés (4), est compris entre 3 et 12 mm, en particulier entre 3,5 et 5,5 mm.

7. Ensemble selon l'une ou plusieurs des revendications précédentes, **caractérisé par le fait que** la matière fibreuse se compose de fibres de coton ou comprend des fibres de coton.

8. Ensemble selon la revendication 7, **caractérisé par le fait que** la matière fibreuse comprend des mélanges de fibres de coton et de fibres discontinues synthétiques ou de fibres discontinues de viscose.

9. Ensemble selon l'une ou plusieurs des revendications précédentes, **caractérisé par le fait que** la tête ouatée est réalisée à partir d'un non-tissé ou d'un ruban de non-tissé d'un poids spécifique compris entre 0,5 et 8 g/m, en particulier entre 1 et 2 g/m.

10. Ensemble selon la revendication 9, **caractérisé par le fait que** le non-tissé ou le ruban de non-tissé est un voile de carde ayant une orientation s'étendant dans la direction de la machine.

11. Procédé de fabrication d'un ensemble selon l'une ou plusieurs des revendications précédentes, comprenant les étapes de procédé suivantes:
- fournir ledit récipient d'emballage (6),
- doser une quantité prédéterminée de liquide de mouillage sur la surface intérieure de fond (24) du récipient d'emballage (6),
- fournir la pluralité de bâtonnets ouatés (4) avec un marquage sous forme d'une coloration insoluble par le liquide de mouillage, de la matière fibreuse d'une seule (20) des têtes ouatées (20, 22),
- mettre en place les bâtonnets ouatés (4) dans le récipient d'emballage (6) de telle manière que les têtes ouatées (20) marquées soient appuyées directement contre la surface intérieure de fond (24) et
- fermer le récipient d'emballage (6) par ledit couvercle (16).

12. Procédé selon la revendication 11, **caractérisé par le fait que** la quantité dosée de liquide de mouillage est comprise entre 2,5 et 6,5 g, en particulier entre 3 et 6 g et encore en particulier entre 4 et 5 g de liquide par 50 pièces de bâtonnets ouatés.
